Europäisches Patentamt

(19) European Patent Office

Office européen des brevets

(11) Veröffentlichungsnummer: **0 064 302**
B1

(12) **EUROPÄISCHE PATENTSCHRIFT**

(45) Veröffentlichungstag der Patentschrift:
24.10.84

(21) Anmeldenummer: 82103898.1

(22) Anmeldetag: 05.05.82

(51) Int. Cl.³: **C 07 C 103/52**, A 61 K 37/02 //
A61K35/38, A61K35/55,
A61K35/60

(54) **Neues Peptid, Verfahren zu seiner Gewinnung und dieses enthaltendes Arzneimittel.**

(30) Priorität: 06.05.81 DE 3117934

(43) Veröffentlichungstag der Anmeldung:
10.11.82 Patentblatt 82/45

(45) Bekanntmachung des Hinweises auf die Patenterteilung:
24.10.84 Patentblatt 84/43

(84) Benannte Vertragsstaaten:
AT BE CH DE FR GB IT LI LU NL SE

(56) Entgegenhaltungen:
US - A - 3 928 306

(73) Patentinhaber: **Max-Planck-Gesellschaft zur Förderung
der Wissenschaften e.V., Bunsenstrasse 10,
D-3400 Göttingen (DE)**

(72) Erfinder: **Schaller, Hildegard Chica, Jahnstrasse 21,
D-6900 Heidelberg (DE)**
Erfinder: **Bodenmüller, Heinz, Parkstrasse 39,
D-6700 Ludwigshafen (DE)**

(74) Vertreter: **Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)**

## Beschreibung

Die Erfindung betrifft ein bisher nicht bekanntes Peptid aus 11 Aminosäuren, ein Verfahren zu seiner Gewinnung und seine Verwendung in Arzneimitteln.

Die erfindungsgemässe Peptid besteht aus 11 Aminosäuren und weist folgende Sequenz auf pGlu-Pro-Pro-Gly-Gly-Ser-Lys-Val-Ile-Leu-Phe.

Die oben angegebene Sequenz wurde durch Synthese bestätigt.

Das erfindungsgemässe Peptid weist folgende Retentionszeiten auf einer reversen Phase Octylsilansäule (LiChrosorb® RP-8, Teilchengrösse 7 μm, Säulengrösse 250-4 mm) bei einer Flussrate von 1 ml/min auf:

1) in 50% Methanol in 5 mM Ammoniumbicarbonat isokratisch 7,6 min,

2) mit Gradient (10 min) von 40 auf 60% Methanol in 5 mM Ammoniumbicarbonat 10,5 min,

3) in 30% Acetonitril in 0,1% TFA (Trifluoressigsäure) isokratisch 8 min, und

4) mit Gradient (10 min) von 20 auf 40% Acetonitril in 0,1% TFA 11,2 min.

Das erfindungsgemässe Peptid stellt die wirksame Substanz des sogenannten Kopfaktivators dar. Bei Hydra wirkt die Verbindung als Mitogen und ist dort für die Zellteilung aller Zelltypen nötig. Bei Hydra ist die Verbindung ausserdem verantwortlich für die kopfspezifische Determination, d.h. eine interstitielle Stammzelle wird durch die Verbindung zur Nervenzelle determiniert. Diese Wirkungen treten bei sehr geringen Konzentrationen in der Grössenordnung von $10^{-13}$M auf. Auf Säugetiere hat die Verbindung eine wachstumsstimulierende Wirkung auf embryonale Hirnzellen, und sie wirkt hypertensiv (pressorisch) bei intraventrikulärer Applikation. Aufgrund ihrer biologischen Eigenschaften ist die erfindungsgemässe Verbindung eine interessante Forschungschemikalie und eröffnet darüber hinaus interessante Verwendungsmöglichkeiten als Arzneimittel.

Es wurde gefunden, dass sich die erfindungsgemässe Verbindung aus tierischen Geweben, insbesondere aus der Hypothalamusregion des Gehirns oder aus dem Darm von Säugetieren oder aus ganzen Coelenteraten gewinnen lässt. Das erfindungsgemässe Verfahren zu ihrer Gewinnung ist dadurch gekennzeichnet, dass man das tierische Gewebe mit einem organischen Lösungsmittel (wie z.B. Aceton, Essigsäure oder Methanol) extrahiert, zentrifugiert und den konzentrierten (z.B. durch Ultrafiltration) Extrakt durch Chromatographie über Ionenaustauscher (z.B. schwach saure Anionen- oder schwach basische Kationenaustauscher) und über schwach vernetzte Molekularsiebsäulen (z.B. Dextran oder Polyacrylamid) reinigt und die das gesuchte Peptid enthaltenden Fraktionen dann der Hochdruckflüssigkeitschromatographie (z.B. über reverse Phase Octyl- oder Actadecasilylsäulen) unterwirft.

Nach diesem Verfahren lassen sich aus 200 kg Seeanemonen bzw. aus 3000 Rattendärmen jeweils 20 bis 30 nmol der Verbindung in Reinform gewinnen. Aus einem menschlichen Hypothalamus ergibt dieses Verfahren etwa 1 nmol der Verbindung. Durch die nach üblichen Methoden der Peptidsynthese durchführbare Herstellung der Verbindung aus den Aminosäuren wird die Verbindung leicht in beliebiger Menge zugänglich.

Die Synthese der erfindungsgemässen Verbindung kann über alle Peptidsyntheseverfahren erfolgen, z.B. nach der Festphasensynthese nach Merrifield mit symmetrischen Anhydriden [Hoppe Seyler's „Z. Physiol. Chem.", *353*, 1973-1976 (1972)] oder in flüssiger Phase mit gemischten Anhydriden [Liebigs „Ann. Chem.", *572*, 190-194 (1951)] mit oder ohne Verwendung von Peptidsyntheseautomaten.

Eine Synthese über Klonierung der kodierenden Nukleinsäure ist auch möglich.

Die Verbindung enthält, wie die Formel zeigt, je 2 Moleküle Prolin und Glycin sowie 7 weitere Aminosäuren jeweils einmal. Eine für viele Peptidhormone charakteristische Besonderheit ist das Vorliegen einer Pyroglutaminsäure am Aminoende. Das Molekül zeichnet sich durch starke Hydrophobizität im carboxyterminalen Teil aus.

Bei der Gewinnung aus tierischem Ausgangsmaterial wird letzteres im organischen Lösungsmittel (z.B. Methanol) möglichst fein zerkleinert, wobei eine niedrige Temperatur eingehalten werden muss, die 10°C nicht übersteigen soll. Nach Zentrifugation wird der konzentrierte Überstand mit Petroläther und Chloroform gewaschen, um Lipide abzutrennen. Die folgenden Chromatographieschritte werden vorzugsweise bei etwa neutralem pH durchgeführt. Wahlweise kann auch eine Behandlung mit einem schwach basischen Anionenaustauscher, wie einem Diäthylamino-äthyl-Gruppen tragenden vernetzten Dextran vorgeschaltet werden, wobei die erfindungsgemässe Verbindung bei 5 mM Ammoniumacetat bei pH <7,5 gebunden und bei pH <3,5 wieder eluiert. Als schwach vernetztes Dextrangel hat sich, wegen seiner atypischen Adsorptionseigenschaften, besonders das unter der Bezeichnung Sephadex G-10 im Handel erhältliche Produkt bewährt. Als Polyacrylamidgel erwies sich das im Handel unter der Bezeichnung Biogel® P 2 erhältliche Produkt als besonders gut geeignet. Jedoch können auch andere Dextrangel bzw. Polyacrylamidgelpräparate verwendet werden, welche vergleichbare Eigenschaften, insbesondere hinsichtlich des Vernetzungsgrades, aufweisen.

Die Elution erfolgt mit verdünnter Salzlösung, beispielsweise 0,1M NaCl. Der gewünschte pH-Wert im angegebenen Bereich kann mit beliebigen Puffersubstanzen eingestellt werden. Bevorzugt wird Trispuffer.

Für die Hochdruckflüssigkeitschromatographie wird als Trägermaterial das reverse Phase Actylsilan, das im Handel unter der Bezeichnung LiChrosorb® RP-8 erhältlich ist, bevorzugt. Auf diesem Trägermaterial wird die erfindungsgemässe Verbindung unter polaren Bedingungen (wenig organische Komponente wie z.B. Methanol oder Acetonitril in der mobilen Phase) zurückgehalten und sie wird bei apolaren Bedingungen (mit viel organischer Komponente in der mobilen Phase) eluiert.

Die synthetische Herstellung der erfindungsgemässen Verbindung kann, wie oben schon erwähnt, nach den üblichen Methoden der Proteinsynthese erfolgen. Die aminoendständige Pyroglutaminsäure-Gruppe lässt sich durch Einbau, z.B. von Glutamin und anschliessender Zyklisierung durch Erhitzen in Säure (z.B. TFA) bilden.

Aufgrund der oben beschriebenen Eigenschaften der erfindungsgemässen neuen Verbindung stellt dieses Neuropeptid, das als erstes im Tierreich vorkommt und bis zum Menschen strikt seine Struktur konserviert hat, eine interessante Forschungssubstanz dar und kann als Wirkstoff in Arzneimitteln zur Wachstumsregulierung und/oder als Transmittersubstanz verwendet werden.

Die folgenden Beispiele erläutern die Erfindung weiter.

*Beispiel 1:*

10 bis 13 kg tiefgefrorene *Anthopleura elegantissima*, Pacific Biomarine Laboratories, California, werden in 1 bis 3 cm kleine Stücke gehackt, kaltes Methanol (−20°C) wird zugegeben, bis die Stücke gut bedeckt sind (ungefähr 1 Teil Stücke zu 2 Teile Methanol). Das erhaltene Gemisch wird homogenisiert (Ultraturax). Die Temperatur sollte dabei 10°C nicht überschreiten (mit Eisbad, techn. NaCl oder Viehsalz kühlen). Die erhaltenen Homogenate werden bei 2000 tr/min 10 min zentrifugiert (Rotor und Zentrifuge vorkühlen, bei 4°C laufen lassen). Die Überstände werden gesammelt und bei −20°C aufbewahrt. Die Sedimente werden im Zentrifugenbecher mit kaltem (−20°C) Methanol aufgelöst und Homogenisieren und Zentrifugieren 2mal wiederholt. Die vereinigten Überstände werden im Rotationsverdampfer auf 10 bis 15 l eingeengt und mit kaltem Petroläther geschüttelt. Die Petrolätherphase wird verworfen. Die Wäsche wird wiederholt, bis die grüne Farbe im Äther ist. Die gewaschene Lösung wird 3mal mit kaltem Chloroform extrahiert, ebenso 1mal die erste Chloroformphase. Die gesammelten Wasserphasen werden auf ein möglichst geringes Volumen (Rotationsverdampfer) eingeengt, eingefroren und getrocknet. Der gefriergetrocknete Rückstand wird mit möglichst wenig Methanol aufgelöst, zentrifugiert und der Überstand gewonnen. Das Sediment wird nochmals mit Methanol extrahiert und zentrifugiert. Die gesammelten Überstände werden evaporiert, in dest. $H_2O$ aufgelöst (Gesamtvolumen etwa 500 ml), auf pH 7,6 gebracht und je 250 ml auf eine Sephadex G-10 Säule (5,3 l Gesamtvolumen, 10 × 73,5 cm) aufgetragen.

Alternativ werden auf 500 ml Extrakt 100 g Sephadex® DEAE-A-25, über Nacht mit 5 mM $NH_4Ac$, pH 7,6, äquilibriert, mit dem Überstand 30 min geschüttelt und 30 min bei 1500 tr/min zentrifugiert. Der Überstand wird auf neues DEAE gegeben (100 g) und dies so oft wiederholt, bis keine Farbe mehr an DEAE-Sephadex® bindet (normalerweise 3- bis 4mal. Das DEAE-Sephadex® wird mit 5 mM $NH_4Ac$, pH 3,5, eluiert, bis keine Farbe mehr im Überstand ist. Das Eluat wird neutralisiert, eingeengt und je 25 ml werden auf eine Sephadex G-10 Säule (Gesamtvolumen 250 ml, 5,5 × 13 cm) aufgetragen und mit dest. $H_2O$ chromatographiert.

Nun werden die Fraktionen 9,5 bis 14,5 l der 5,3 l G-10 Säule oder die Fraktionen 500 bis 1000 ml der 250 ml G-10 Säule auf 20 ml eingeengt und auf eine Acrylamidgelsäule (Biogel® P-2; 660 ml; 4,5 × 50 cm) gegeben und mit 5 mM Ammoniumbicarbonatlösung eluiert. Die Fraktionen 300 bis 450 ml werden vereinigt, auf 1 ml eingeengt und auf eine weitere G-10 Säule (Volumen 25 ml; 1,5 × 16 cm) gegeben und mit 0,1M NaCl, 0,01M Tris-HCl, pH 7,6, eluiert. Die vereinigten Fraktionen 29 bis 42 ml werden auf 1 ml eingeengt und auf eine Acrylamidgelsäule (Biogel® P-2, 40 ml; 1,3 × 47 cm) gegeben und mit dem gleichen Elutionsmittel wie im vorhergehenden Schritt eluiert.

Die aktiven Fraktionen werden vereinigt, auf 0,5 ml eingeengt und in eine Pasteurkapillarpipette gegeben, die 0,5 ml LiChrosorb® RP-8, Teilchengrösse 10 µm enthält, welches mit 20% Methanol in 5 mM Ammoniumbicarbonat äquilibriert ist. Nach Waschen mit dem Äquilibrierungsmittel wird mit 3 ml 80%igem Methanol in 5 mM Ammoniumbicarbonatlösung eluiert, das Eluat zur Trokkene gebracht, in 0,1% TFA wieder aufgenommen und die Behandlung in der Pasteurkapillarpipette wiederholt, wobei jedoch als Elutionsmittel 3 ml 40%iges Acetonitril in 0,1% TFA verwendet werden. Das Eluat wird eingeengt und auf eine Hochdruckflüssigkeitschromatographiesäule (LiChrosorb® RP-8, Teilchengrösse 7 µm; 250 × 4 mm) gegeben, die mit 20% Acetonitril in 0,1% TFA äquilibriert wurde und mit einem Gradienten bis 40% Acetonitril in 0,1% TFA 10 min eluiert bei einer Fliessgeschwindigkeit 1 ml/min. Die Retentionszeit der erfindungsgemässen Verbindung beträgt 10,6 min.

Das Eluat wird auf 50 µl konzentriert und erneut auf eine gleiche Hochdruckflüssigkeitschromatographiesäule gebracht, die mit 40%igem Methanol in 5 mM Ammoniumbicarbonat äquilibriert ist und mit einem Gradienten bis 60% Methanol 10 min eluiert. Fliessgeschwindigkeit 1 ml/min. Die Retentionszeit der erfindungsgemässen Verbindung beträgt 10,5 min. Man erhält so 1 nmol reine Verbindung.

*Beispiel 2:*

1000 Ratten (2 bis 3 Monate alt) werden mit $CO_2$ narkotisiert, die Bauchdecke geöffnet und der Darm ohne Pancreas vom Pförtner an bis zur letzten Darmverdickung vor dem After herauspräpariert. Die Därme werden in 20 l kaltem Methanol gesammelt, mit 0,05% Phenylmethylsulfonylfluorid versetzt und dann, wie in Beispiel 1 beschrieben, weiter aufgearbeitet. Man erhält 10 nmol der erfindungsgemässen Verbindung.

*Beispiel 3:*

Ein menschlicher Hypothalamus (20 h nach dem Tod) wurde bei 4°C mit überschüssigem Methanol homogenisiert und danach 2mal je 2 min mit Ultraschall behandelt. Durch Zentrifugieren wurde ein Überstand erhalten, der nach Einengen mit Petroläther und mit Chloroform gewaschen wurde. Nach Reextraktion mit Wasser wie in Beispiel 1 beschrieben, wurde die wässerige Phase auf 10 ml eingeengt und auf eine Sephadex® G-10 Säule mit 250 ml Volumen wie in Beispiel 1 beschrieben, aufgetragen und eluiert. Die Fraktion 500 bis 1000 ml wurde eingeengt und direkt auf eine LiChrosorb® RP-8 Säule von 0,5 ml aufgetragen. Die weitere Reinigung erfolgt wie in den letzten Stufen von Beispiel 1. Man erhält 1 nmol der erfindungsgemässen Verbindung.

## Patentansprüche

1. pGlu-Pro-Pro-Gly-Gly-Ser-Lys-Val-Ile-Leu-Phe.

2. Verfahren zur Gewinnung der Verbindung von Anspruch 1, dadurch gekennzeichnet, dass man das tierische Gewebe, insbesondere aus der Hypothalamusregion des Gehirnes oder aus dem Darm von Säugetieren oder aus ganzen Coelenteraten, mit einem organischen Lösungsmittel extrahiert, zentrifugiert und den konzentrierten Extrakt durch Chromatographie über Ionenaustauscher und über schwach vernetzte Molekularsiebsäulen reinigt und die das gesuchte Peptid enthaltenden Fraktionen dann der Hochdruckflüssigkeitschromatographie unterwirft.

3. Arzneimittel, gekennzeichnet durch einen Gehalt der Verbindung von Anspruch 1 als Wirkstoff zusammen mit üblichen pharmazeutischen Konfektionierungs- und Verdünnungsmitteln.

## Revendications

1. pGlu-Pro-Pro-Gly-Gly-Ser-Lys-Val-Ile-Leu-Phe.

2. Procédé d'obtention du composé selon la revendication 1, caractérisé en ce qu'on extrait, avec un solvant organique, le tissu animal, en particulier de la région de l'hypothalamus du cerveau ou de l'intestin de mammifères ou de cœlentérés entiers, en ce qu'on centrifuge et purifie l'extrait concentré par chromatographie sur échangeur d'ions et sur des colonnes de tamis moléculaire faiblement réticulé, puis en ce qu'on soumet les fractions renfermant le peptide recherché à une chromatographie liquide sous haute pression.

3. Médicament, caractérisé en ce qu'il renferme le composé selon la revendication 1 en tant que principe actif, avec des agents de dilution et de formulation pharmaceutiques habituels.

## Claims

1. pGlu-Pro-Pro-Gly-Gly-Ser-Lys-Val-Ile-Leu-Phe.

2. Process for obtaining the compound of Claim 1, characterised in that one extracts the animal tissue, especially from the hypothalamus region of the brain or from the intestine of mammalians or from whole coelenterata, with an organic solvent, centrifuges and purifies the extract by chromatography over ion exchangers and over weakly cross-linked molecular sieve columns and then subjects the fractions containing the desired peptide to high-pressure liquid chromatography.

3. Medicament, characterised by a content of the compound of Claim 1 as active material, together with conventional pharmaceutical confectioning and diluting agents.